# FASCICULE DE BREVET EUROPEEN

(11) **EP 0 847 294 B1**
(45) Date de publication et mention de la délivrance du brevet: **23.07.2003**
(21) Numéro de dépôt: 96930193.6
(22) Date de dépôt: 30.08.1996
(51) Int. Cl.: A61N 1/32, A61N 1/34

(54) **DISPOSITIF DE SOULAGEMENT DES DOULEURS PAR APPLICATION D'IMPULSIONS ELECTRIQUES**
SCHMERZLINDERUNGSVORRICHTUNG MITTELS ELEKTRISCHEN IMPULSEN
PAIN RELIEF DEVICE THROUGH APPLICATION OF ELECTRIC PULSES

(30) Priorité: 31.08.1995 FR 9510406
(43) Date de publication de la demande: 17.06.1998
(73) Titulaire: Dervieux, Dominique, 06300 Nice (FR)
(72) Inventeur: Dervieux, Dominique, 06300 Nice (FR)
(74) Mandataire: Bonneau, Gérard
(86) Numéro de dépôt international: FR9601338
(87) Numéro de publication internationale: WO97007855

(56) Documents cités:
- EP-A- 0 603 451
- FR-A- 1 137 482
- FR-A- 2 621 827
- FR-A- 2 624 748
- US-A- 4 175 551
- US-A- 5 514 167

## Description

### Domaine technique

La présente invention concerne de façon générale les dispositifs permettant de soulager les douleurs au moyen d'impulsions électriques appliquées sur la peau de l'utilisateur, et en particulier un dispositif de ce type présentant une grande efficacité et sans gêne pour l'utilisateur quelle que soit la position du dispositif par rapport à la peau.

### Etat de la technique

On connaît déjà des dispositifs de soulagement des douleurs par application d'impulsions électriques, dans lesquels le courant électrique est produit par une décharge d'origine piézo-électrique. La demande de brevet EP-O214136 au nom de Dervieux, publiée le 18.03.87, décrit un appareil de soulagement des douleurs par des étincelles d'origine piézo-électrique. La tête d'application est constituée par une électrode bipolaire, avec soit un pôle au contact direct de la peau et l'autre pôle à une certaine distance de la peau, soit les deux pôles à une certaine distance de la peau. Lors de la compression du cristal piézo-électrique, une décharge d'électrons chauds d'électricité statique se produit pour aller frapper la peau, avec des effets bénéfiques.

Selon un autre type d'appareils, les décharges électriques ne sont pas produites par des éléments piézo-électriques, mais par un générateur du type « à relaxation ». La demande de brevet FR-2624748 publiée le 23.06.89 au nom de Kogan, décrit un tel type d'appareil, selon lequel les décharges électriques qui s'appliquent sur la peau de l'utilisateur, sont produites par un générateur de type « R.C. ».

D'autres dispositifs du même type, également basés sur la production d'impulsions électriques appliquées sur la peau de l'utilisateur, sont décrits dans les brevets FR-A-2.621.827, EP-A-0.603.451 et EP-A-0.387.176.

Tous ces dispositifs comportent une tête qui doit être maintenue en contact avec la peau lors de l'application d'impulsions électriques. Mais la surface de la peau n'étant pas plate, il est très difficile pour l'utilisateur de maintenir en permanence la tête en contact avec la peau. La moindre inclinaison de la tête augmente la distance séparant les électrodes de la peau, fait augmenter la tension et l'intensité des impulsions, surtout lorsque la peau est mouillée ou dans une atmosphère humide. Ainsi, un dixième de millimètre d'écartement entraîne une augmentation de 100 volts, un millimètre une augmentation de 1000 volts. Cette tension est doublée si les deux pôles de l'électrode bipolaire sont en retrait de la tête d'application. Enfin, ces dispositifs donnent des décharges intempestives, en particulier en approchant et en retirant l'appareil.

Les électrodes utilisées dans les dispositifs décrits dans les documents cités ci-dessus ont généralement une surface d'application complexe avec de nombreuses excavations favorisant l'accumulation de diverses salissures dangereuses sur le plan hygiénique et risquant d'amener des infections. De plus, il est difficile d'y coller une protection hygiénique renouvelable.

Le dispositif qui fait l'objet du brevet US 4.175.551 remédie partiellement à ces inconvénients puisqu'il divulgue un dispositif portatif de soulagement des douleurs comprenant une tête d'application d'impulsions de courant électrique sur la peau d'un utilisateur et une poignée tenue dans la main de l'utilisateur, la tête ayant la forme d'une portion de cylindre dont la directrice est de forme convexe et comprenant au moins une électrode qui se trouve sur la surface extérieure du cylindre, et la tête étant toujours en contact avec la peau de l'utilisateur quelle que soit sont inclinaison. Enfin, de façon à utiliser l'appareil sur des peaux humides, une des électrodes doit être mise directement en contact avec la peau et l'autre à une distance suffisante d'environ un millimètre (soit une tension d'environ 1000 volts) pour qu'elle ne touche pas la peau sinon cela annulerait son efficacité ; il y a donc là une limite inférieure élevée du voltage d'utilisation de ce type d'appareil.

Tout ceci concourt à une utilisation douloureuse et imprévisible de ces appareils, en particulier en raison de l'augmentation de l'intensité selon l'inclinaison de leur tête plate par rapport à la peau, de la présence de décharge intempestives, de la mauvaise répartition des décharges et donc de son efficacité, entraînant chez l'utilisateur des réactions de crainte et le plus souvent de rejet.

### Exposé de l'invention

C'est pourquoi le but de l'invention est de fournir un dispositif de soulagement par impulsions électriques ne présentant pas les inconvénients ci-dessus quelle que soit sa position d'utilisation.

Un autre but de l'invention est de fournir un dispositif de soulagement par application d'impulsions électriques de tension constante sur la peau, que la peau soit sèche ou humide et quel que soit l'angle d'inclinaison de la tête d'application par rapport à la peau.

L'objet de l'invention est donc un dispositif portatif de soulagement des douleurs et contractures et de régénérescence cutanée comprenant une poignée tenue dans la main de l'utilisateur et une tête d'application d'impulsions de courant électrique sur la peau d'un utilisateur ayant la forme d'une portion de cylindre dont la directrice est de forme convexe. La tête d'application des impulsions de courant électrique comprend des électrodes pour la génération des impulsions, se trouvant à la surface extérieure du cylindre ou à une distance constante de cette surface extérieure et dont au moins une portion se trouve située sur chaque génératrice du cylindre susceptible d'être en contact avec la peau de l'utilisateur, la tête restant toujours en contact avec la peau de l'utilisateur par une des génératrices de la portion de cylindre comportant la portion d'électrode quelle que soit l'inclinaison de la tête par rapport à la peau de l'utilisateur. Les électrodes sont composées d'un premier ensemble d'électrodes parallèles formant un peigne connecté à une même borne de polarité donnée à un instant donné et un second ensemble d'électrodes parallèles formant un peigne connecté à une même borne de polarité opposée au même instant donné, les électrodes du second ensemble étant alternées avec les électrodes du premier ensemble et les électrodes des deux ensembles étant perpendiculaires aux génératrices de la tête en forme de portion de cylindre de sorte qu'une portion de chacune des électrodes est toujours en contact ou à une distance constante de la peau le long de la génératrice de la portion de cylindre en contact avec la peau quelle que soit l'inclinaison de la tête par rapport à la peau de l'utilisateur.

Selon une caractéristique de l'invention, la tête d'application des impulsions de courant électrique est recouverte d'une couche d'isolant telle qu'un sparadrap poreux non tissé, d'épaisseur constante, de telle sorte que les électrodes se trouvent séparées de la peau de l'utilisateur par une distance constante, par exemple inférieure à 1 mm, quelle que soit l'inclinaison de la tête par rapport à la peau.

La configuration du dispositif selon l'invention rend possible son utilisation sur toutes les parties cutanées de l'utilisateur malgré des angles d'application qui peuvent varier jusqu'à presque 180° par rapport à la surface de la peau, ceci sans variation d'intensité et décharge intempestive.

Un tel dispositif présente de nombreux avantages et en particulier il permet, d'une part d'utiliser des tensions minimales, bien inférieures aux tensions des appareils antérieurs qui utilisaient plusieurs milliers de volts tandis que quelques centaines de volts suffisent avec ce type d'appareil ; et d'autre part de limiter et de régulariser électroniquement l'intensité maximum des décharges avant la limite de la douleur et supprimer ainsi les décharges intempestives.

### Description brève des figures

Les buts, objets et caractéristiques de la présente invention ressortiront plus clairement à la lecture de la description qui suit faite en référence aux dessins dans lesquels :
la figure 1 représente une vue en perspective d'un mode de réalisation préféré du dispositif selon l'invention,
la figure 2 représente une coupe de la tête d'application présentant une inclinaison de 60° par rapport à l'axe de la poignée,
la figure 3 montre la face avant d'une tête d'application comportant des électrodes parallèles, de polarité alternée, perpendiculaires à la génératrice de la portion de cylindre,
la figure 4 représente schématiquement une vue en perspective de la tête dont la face avant est illustrée sur la figure 3,
la figure 5 représente une coupe de la tête selon l'invention en contact avec la peau de l'utilisateur,
la figure 6 représente la coupe d'une tête d'application dont les électrodes sont recouvertes d'une couche d'un matériau isolant, et
la figure 7 est le schéma d'un mode de réalisation préféré du circuit électronique utilisé pour obtenir les impulsions de courant électrique dans le dispositif selon l'invention.

### Description détaillée de l'invention

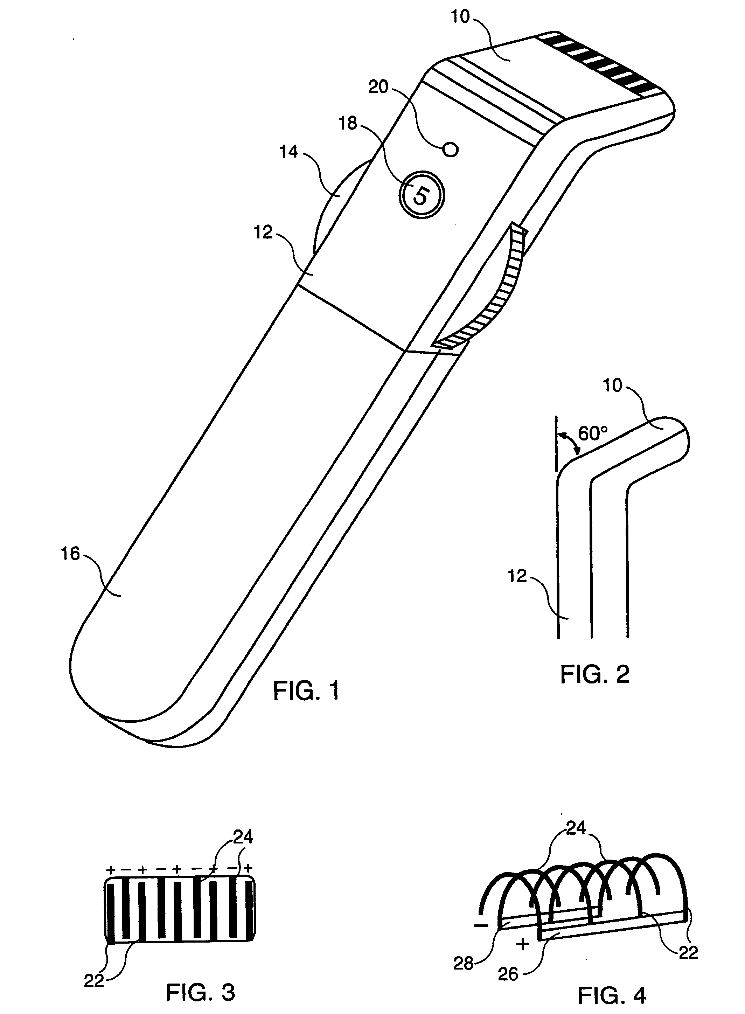

Un premier mode de réalisation du dispositif selon l'invention représenté sur la figure 1 est constitué d'une tête 10 d'application des impulsions de courant électrique sur la peau de l'utilisateur, d'un corps 12 de forme allongée et aplatie, comportant un commutateur 14 pour la mise en marche et l'arrêt du dispositif et une poignée 16 tenue par l'utilisateur. Dans le corps 12, une fenêtre 18 laisse apparaître un chiffre indiquant le niveau de tension de l'appareil, par exemple 0, 1, 2, 3, 4, 5; et une diode luminescente 20 indique que le dispositif est allumé.

Comme on le voit sur la figure 2, la tête 10 est inclinée par rapport au corps 12 d'un angle d'environ 60° de façon à faciliter le positionnement du dispositif sur la peau lorsqu'il est tenu par l'utilisateur. La tête 10 a la forme d'une portion de cylindre dont la directrice est de préférence circulaire mais peut avoir une tout autre forme mais toujours convexe.

Dans un mode de réalisation préféré, la tête comporte des électrodes de polarité opposée alternées, par exemple des électrodes positives 22 (à un instant donné) alternées avec des électrodes négatives 24 (au même instant) comme illustré sur la figure 3. Les électrodes 22 et 24 sont parallèles entre elles, perpendiculaires aux génératrices de la tête cylindrique et séparées par une distance d'environ de 1 à 3 mm.

Les électrodes se présentent en perspective de la manière illustrée sur la figure 4, toutes les électrodes positives 22 à un instant donné étant connectées à la borne positive 26 et formant ainsi un peigne, et toutes les électrodes négatives 24 au même instant étant connectées à la barre négative 28 et formant également un peigne.

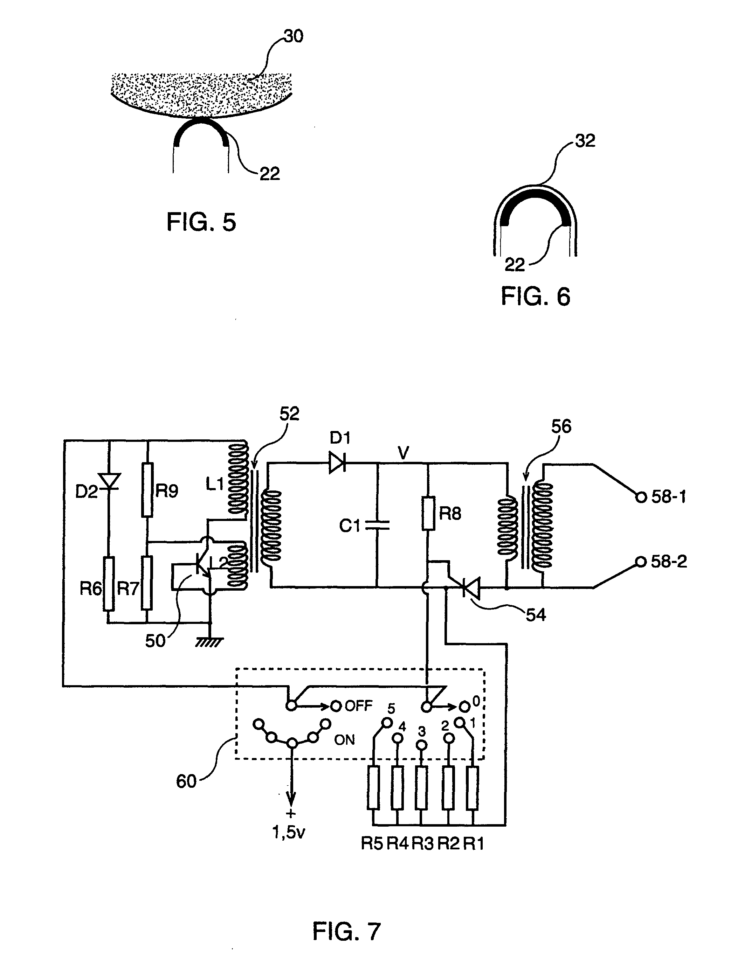

La figure 5 illustre une vue en coupe de la tête, les électrodes 22 (ou 24) étant en contact avec la peau 30 de l'utilisateur. Cette disposition en arc de cercle des électrodes et l'application de la tête cylindrique par une génératrice assure un contact permanent des électrodes sur la peau même si la tête prend une inclinaison importante avec la verticale de la peau et qui peut atteindre presque un angle droit de part et d'autre.

Dans une variante du mode de réalisation précédent, les électrodes 22 et 24 peuvent être recouvertes d'une couche 32 d'un matériau isolant d'une épaisseur constante tel qu'illustré sur la figure 6. Un tel matériau qui assure hygiène et protection peut être par exemple du sparadrap poreux non tissé. L'épaisseur de la couche de protection ne doit pas dépasser 1 mm si on veut éviter d'avoir à fournir des tensions trop élevées.

Que ce soit lorsque les électrodes sont en contact direct avec la peau ou bien s'en trouvent séparées par un matériau isolant de protection hygiénique d'épaisseur constante ou même un vêtement, le principe fondamental du dispositif qui vient d'être décrit est l'application dans la peau d'impulsions électriques d'une fréquence fixe comprise entre 10 et 150 Hertz et une tension constante inférieure à 2000 volts.

Ces impulsions doivent provoquer des sensations de picotement d'une intensité maximale (variable selon les individus) pour pouvoir être efficaces, sans toutefois arriver au seuil de la douleur. Le mécanisme d'action physiologique de ce type de dispositif est décrit dans le brevet français 84 07087.

La génération des impulsions de courant électrique peut se faire de deux façons. Dans un premier mode, le générateur est de type piézo-électrique et est commandé manuellement. Il peut alors comprendre un éclateur ou condensateur de décharge constitué d'une rupture de continuité de l'isolant du fil électrique haute tension allant à la tête du dispositif, en mettant le fil métallique à nu et à une distance d'un 1/10 de mm à quelques mm de l'étrier métallique de génération constituant l'autre pôle.

La génération des impulsions peut également être produite par un circuit électronique du type de celui décrit sur la figure 7. Dans ce circuit, le transistor 50 est utilisé comme commutateur connectant alternativement une inductance L₁ formant un premier enroulement primaire d'un transformateur 52 à une source de tension de 1,5 volts qui peut être une pile se trouvant dans la poignée du dispositif. Le transformateur 52 fournit une tension de réaction induite dans l'inductance L₂ formant un second enroulement primaire qui est appliquée à la base du transistor 50. Du fait que la diode D₁ du secondaire est polarisée en inverse, le secondaire du transformateur 52 est un circuit ouvert, et par conséquent, la variation d'intensité dans L₁ est pratiquement linéaire pendant la période de conduction du transistor 50. Pendant cette période, le courant collecteur du transistor 50 augmente jusqu'à sa valeur maximale, imposée par le courant de base correspondant à une valeur d'induction inférieure à la saturation. A ce moment, la variation du courant collecteur diminue (c'est à dire que la dérivée di/dt→0) et la tension induite dans l'enroulement L₂ diminue pendant que la résistance équivalente du transistor 50 devient très grande. L'effet étant cumulatif, il y a pratiquement coupure brusque de l'intensité collecteur dans l'enroulement primaire. Il est à noter que les résistances R₆, R₇, R₉ ainsi que la diode D₂ sont des éléments classiques pour ce type de circuit et dont le rôle est d'établir des chemins d'écoulement du courant.

Le changement du sens de variation du courant dans le primaire du transformateur 50 provoque une inversion du sens de la tension aux bornes de l'enroulement secondaire. La diode D₁ devient conductrice. L'énergie électromagnétique ½.L.I² emmagasinée pendant la première partie du cycle est reportée au secondaire dans le condensateur C₁ qui se charge. Le circuit a ainsi joué le rôle d'un convertisseur de tension continue en tension alternative.

Dans la deuxième partie du circuit de la figure 7, un thyristor 54 a sa gâchette connectée à la borne d'une résistance R₈ faisant partie d'un pont diviseur, ce qui modifie la tension à atteindre sur le condensateur C₁ pour déclencher le thyristor 54. Lorsque cette tension V est atteinte, le thyristor 54 devient conducteur et la capacité C₁ se décharge dans l'enroulement primaire d'un transformateur haute tension 56, ce qui provoque une impulsion dans le secondaire de tension plus ou moins importante suivant le rapport de transformation du transformateur et fournie entre les bornes de sortie 58-1 et 58-2 connectées aux électrodes du dispositif.

Le commutateur 60 à cinq positions plus la position déconnectée 0, permet de connecter la tension +1,5 volts par l'intermédiaire des résistances R₁, R₂, R₃, R₄, et R₅ de valeur croissante. La valeur de la résistance connectée détermine le niveau de tension V atteint en un temps variable selon cette valeur, et donc fait varier la fréquence des impulsions fournies entre les bornes 28, en même temps que la tension. On doit noter que, plus la tension de sortie augmente (lorsqu'on passe de R₁ à R₅), plus la fréquence des impulsions diminue et réciproquement.

Bien que dans le mode de réalisation ci-dessus, la tension de sortie du dispositif ne puisse prendre que cinq valeurs, il est possible de prévoir un plus grand nombre de résistances et donc de valeurs. Mais il est également possible de remplacer les résistances discrètes par un rhéostat permettent de faire varier la tension de sortie de façon continue.

## Revendications

1. Dispositif portatif de soulagement des douleurs et contractures et de régénérescence cutanée comprenant une tête d'application d'impulsions de courant électrique (10) sur la peau d'un utilisateur et d'une poignée (16) tenue dans la main de l'utilisateur, ladite tête ayant la forme d'une portion de cylindre dont la directrice est de forme convexe et comprend des électrodes (22, 24) pour la génération des impulsions de courant électrique et se trouvant à la surface extérieure dudit cylindre ou à une distance constante de cette surface extérieure et dont au moins une portion se trouve située sur chaque génératrice dudit cylindre susceptible d'être en contact avec la peau de l'utilisateur, ladite tête étant toujours en contact avec la peau de l'utilisateur par une des génératrices de ladite portion de cylindre comportant ladite portion d'électrodes quelle que soit l'inclinaison de ladite tête par rapport à la peau de l'utilisateur ;
ledit dispositif étant **caractérisé en ce que** lesdites électrodes comprennent un premier ensemble d'électrodes (22) parallèles formant un peigne connecté à une même borne (26) de polarité donnée à un instant donné et un second ensemble d'électrodes (24) parallèles formant un peigne connecté à une même borne (28) de polarité opposée au même instant donné, les électrodes dudit second ensemble étant alternées avec les électrodes dudit premier ensemble et les électrodes des deux ensembles étant perpendiculaires aux génératrices de ladite tête (10) en forme de portion de cylindre de sorte qu'une portion de chacune des électrodes est toujours en contact ou à une distance constante de la peau le long de la génératrice de ladite portion de cylindre en contact avec la peau quelle que soit l'inclinaison de ladite tête par rapport à la peau de l'utilisateur.

2. Dispositif selon la revendication 1, dans lequel ladite tête (10) est recouverte d'une couche d'isolant (32) telle qu'un sparadrap poreux non tissé, d'épaisseur constante, de telle sorte que lesdites électrodes se trouvent séparées de la peau de l'utilisateur par une distance constante, par exemple inférieure à 1 mm, quelle que soit l'inclinaison de la tête par rapport à la peau.

3. Dispositif selon la revendication 1 ou 2, dans lequel ladite poignée (16) est reliée à ladite tête d'application des impulsions de courant électrique (10) par un corps (12) dans le prolongement de ladite poignée, ladite tête faisant un angle compris entre 45 et 90° avec l'axe dudit corps.

4. Dispositif selon l'une des revendications 1, 2 ou 3, dans lequel le circuit destiné à la génération des impulsions de courant électrique comprend une source de courant continu (1,5 volt), un convertisseur de tension continue en tension alternative comportant un transistor (50) alternativement conducteur et bloqué et un transformateur (52) fournissant des impulsions de tension alternative, et un transformateur haute tension (56) dont la fréquence et la tension aux bornes de sortie (58) dépend du déclenchement d'un thyristor (54) en fonction du niveau de puissance imposé par un commutateur (60) actionné par l'utilisateur, la tension de sortie augmentant en même temps que la fréquence diminue et réciproquement.

5. Dispositif selon l'une des revendications 1, 2 ou 3, dans lequel le circuit destiné à la génération des impulsions de courant est de type piézo-électrique, commandé manuellement et comprend un éclateur ou condensateur de décharge constitué d'une rupture de continuité de l'isolant du fil électrique haute tension allant à la tête du dispositif, en mettant le fil métallique à nu et à une distance d'un 1/10 de mm à quelques mm de l'étrier métallique de génération constituant l'autre pôle.

## Patentansprüche

1. Tragbare Vorrichtung zum Lindern von Schmerzen und Verkrampfungen und zur Hautregenerierung, umfassend einen Kopf. zum Anlegen von elektrischen Stromimpulsen (10) an die Haut eines Benutzers und einen Griff (16), den der Benutzer in der Hand hält, wobei der Kopf die Form eines Zylinderabschnitts besitzt, dessen Leitkurve von konvexer Form ist, und Elektroden (22, 24) zur Erzeugung der elektrischen Stromimpulse aufweist, die sich auf der Außenfläche des Zylinders oder in einem konstanten Abstand von dieser Außenfläche befinden und von denen mindestens ein Abschnitt auf jeder Erzeugenden des Zylinders gelegen ist, die mit der Haut des Benutzers in Kontakt sein kann, wobei der Kopf, unabhängig von seiner Neigung bezüglich der Haut des Benutzers, über eine der Erzeugenden dieses Zylinderabschnitts, der diesen Elektrodenabschnitt aufweist, immer mit der Haut des Benutzers in Kontakt ist,
wobei diese Vorrichtung **dadurch gekennzeichnet ist, daß** die Elektroden eine erste Gruppe von parallelen Elektroden (22), die einen Kamm bilden, der an einen gemeinsamen Anschluß (26) angeschlossen ist, der zu einem gegebenen Zeitpunkt von einer gegebenen Polarität ist, und eine zweite Gruppe von parallelen Elektroden (24) umfassen, die einen Kamm bilden, der an einen gemeinsamen Anschluß (28) angeschlossen ist, der zum selben gegebenen Zeitpunkt von entgegengesetzter Polarität ist, wobei die Elektroden dieser zweiten Gruppe mit den Elektroden der ersten Gruppe abwechseln und die Elektroden der beiden Gruppen zu den Erzeugenden des Kopfes (10) in Form eines Zylinderabschnitts senkrecht sind, so daß ein Abschnitt jeder der Elektroden immer mit der Haut in Kontakt ist oder sich in einem konstanten Abstand von dieser befindet, und zwar längs der Erzeugenden dieses Zxylinderabschnitts, der, unabhängig von der Neigung des Kopfs bezüglich der Haut des Benutzers, mit der Haut in Kontakt ist.

2. Vorrichtung nach Anspruch 1, bei der der Kopf mit einer Isolationsschicht (32), wie poröses Vliespflaster, von konstanter Dicke bedeckt ist, so daß die Elektroden unabhängig von-der Neigung des Kopfs bezüglich der Haut von dieser Haut durch einen konstanten Abstand von beispielsweise weniger als 1 mm getrennt sind.

3. Vorrichtung nach Anspruch 1 oder 2, bei der der Griff (16) mit dem Kopf zum Anlegen der elektrischen Stromimpulse (10) durch einen in der Verlängerung des Griffs befindlichen Körper (12) verbunden ist, wobei der Kopf mit der Achse des Körpers einen Winkel von 45 bis 90° bildet.

4. Vorrichtung nach einem der Ansprüche 1, 2 oder 3, bei der die Schaltung, die zur Erzeugung der elektrischen Stromimpulse bestimmt ist, eine Gleichstromquelle (1,5 Volt), einen Wandler zur Umwandlung von Gleichspannung in Wechselspannung mit einem abwechselnd leitenden und gesperrten Transistor (50) und einem Wechselspannungsimpulse liefernden Transformator (52) und einen Hochspannungstransformator (56) aufweist, dessen Frequenz und Spannung an den Ausgangsanschlüssen (58) von der Zündung eines Thyristors (54) in Abhängigkeit von dem Leistungspegel abhängt, der durch einen durch den Benutzer betätigten Schalter (60) festgelegt wird, wobei die Ausgangsspannung gleichzeitig damit, daß die Frequenz fällt, steigt, und umgekehrt.

5. Vorrichtung nach einem der Ansprüche 1, 2 oder 3, bei der die Schaltung, die zur Erzeugung der elektrischen Stromimpulse bestimmt ist, eine piezoelektrische Schaltung ist, die manuell gesteuert wird und einen Entlader oder Entladungskondensator aufweist, der aus einer Unterbrechung der Kontinuität der Isolation des zum Kopf der Vorrichtung laufenden elektrischen Hochspannungsdrahts gebildet wird, indem der Metalldraht abisoliert wird und in einem Abstand von 1/10 mm bis einigen mm von dem den anderen Pol bildenden Erzeugungs-Metallbügel angeordnet wird.

## Claims

1. Portable device for relief of pain and contractures and for skin rejuvenation comprising a head (10) for applying electric pulses to the skin of a user and a handle (16) held by the user's hand, said head having the shape of a cylinder portion whose directrix has a convex shape and comprises at least one electrode (22, 24) for generating the electric pulses, located on the outer surface of said cylinder or at a constant distance from this outer surface, and a portion of which, at least, is located on each generatrix of said cylinder likely to be in contact with the user's skin, said head being always in contact with the user's skin through one of the generatrices of said cylinder portion comprising said electrode portion, whatever the tilting angle of the head against the user's skin surface;
said device being **characterized in that** said electrodes comprise a first set of parallel electrodes (22) forming a comb connected to a same terminal (26) having a given polarity at a given time, and said second set of parallel electrodes (24) forming a comb connected to a same terminal (28) having an opposite polarity at the same time, the electrodes of said second set being arranged alternately with the electrodes of said first set and the electrodes of the two sets being perpendicular to the generatrices of said head (10) having the shape of a cylinder portion so that a portion of each electrode is always in contact with the skin or located at a constant distance from the skin along the generatrix of said cylinder portion in contact with the skin, whatever the tilting angle of the head against the user's skin surface.

2. Device according to claim 1, wherein said head (10) is covered with a layer of insulating material (32) such as a constant thickness porous non-woven fabric plaster, so that said electrodes are spaced out from the user's skin by a constant distance, for example lower than 1 mm, whatever the tilting angle of the head against the skin.

3. Device according to claim 1 or 2, wherein said handle (16) is linked to said head (10) for applying electric pulses by means of a body (12) lined up with said handle, said head forming an angle ranging from 45° to 90° with the axis of said body.

4. Device according to one of claims 1, 2 or 3, wherein the circuit meant for generating electric pulses comprises a direct current source (1.5 volt), a DC/AC voltage converter comprising a transistor (50) alternately conductive and blocked and a transformer (52) providing alternative voltage pulses, and a high voltage transformer (56) whose frequency and voltage at the output terminals (58) depend on the triggering of a thyristor (54) according to the power level imposed by a switch (60) actuated by the user, the output voltage increasing when the frequency decreases, and vice versa.

5. Device according to one of claims 1, 2 or 3, wherein the circuit meant for generating electric pulses is of piezoelectric type, manually controlled, and comprises a spark gap or discharge capacitor made up of a continuity break of the insulating material of the high voltage electric wire that goes to the head of the device, obtained by stripping the metallic wire at a distance ranging from 1/10 mm to a few millimeters from the generating metallic stirrup that makes up the other pole.
